# FASCICULE DE BREVET EUROPEEN

(11) **EP 3 153 502 B1**
(45) Date de publication et mention de la délivrance du brevet: **19.12.2018**
(21) Numéro de dépôt: 16201330.4
(22) Date de dépôt: 25.09.2013
(51) Int. Cl.: C07C 329/02

(54) **PROCÉDÉ DE SYNTHÈSE D'UN SEL MINÉRAL DE L'ACIDE PIVALIQUE EN TANT QUE PRODUIT INTERMÉDIAIRE**
SYNTHESEVERFAHREN EINES MINERALSALZES DER PIVALINSÄURE ALS ZWISCHENPRODUKT
METHOD FOR SYNTHESISING A MINERAL SALT OF PIVALIC ACID AS AN INTERMEDIATE PRODUCT

(43) Date de publication de la demande: 12.04.2017
(62) Demande divisionnaire de: 13392006.6
(73) Titulaire: H4 Pharma, 78390 Bois D'Arcy (FR)
(72) Inventeur: BOULEY, Etienne, 78170 La Celle Saint Cloud (FR); LIGONES, Pierre, 98000 Monaco (MC); GOUMAIN, Sophie, 51687 Reims Cedex 2 (FR); BIRONNEAU, Sonia, 51687 Reims Cedex 2 (FR)
(74) Mandataire: Hautier IP

(56) Documents cités:
- GB-A- 2 179 350
- O. NIYOMURA, ET AL.: "Facile synthesis and structure of heavy alkali metal thiocarboxylates: structural comparison with the selenium and tellurium isologues", INORGANIC CHEMISTRY, vol. 38, no. 3, 8 février 1999 (1999-02-08), pages 507-518, XP055104983, American Chemical Society, Washington, DC, US ISSN: 0020-1669, DOI: 10.1021/ic9808091
- K. TANI, ET AL.: "Thioacylsulfanylarsines (RCS<2>)<x>AsPh<3-x>, x = 1-3: synthesis, structures, natural bond order analyses and reactions with piperidine", JOURNAL OF THE CHEMICAL SOCIETY, DALTON TRANSACTIONS, no. 5, 13 février 2001 (2001-02-13), pages 518-527, XP055104982, Royal Society of Chemistry, Cambridge, GB ISSN: 0368-1769, DOI: 10.1039/b008702p
- D. SEYFERTH, ET AL.: "Reactions of in-situ generated acyllithium reagents with carbon disulfide and carbonyl sulfide", TETRAHEDRON LETTERS, vol. 25, no. 25, juin 1984 (1984-06), pages 2623-2626, XP055104981, Elsevier Science Publishers, Amsterdam, NL ISSN: 0040-4039, DOI: 10.1016/s0040-4039(01)81246-6

## Description

La présente invention concerne un procédé de synthèse d'un sel minéral de l'acide thiopivalique. La présente invention trouvera son application dans le domaine pharmaceutique pour la synthèse du principe actif tixocortol pivalate. En particulier dans l'industrie des génériqueurs.

O. Niyomura, et al. (Inorg. Chem., 1999, 38, 507-518) divulgue la préparation du sel de césium de l'acide thiopivalique par réaction entre l'acide thiopivalique et le fluorure de césium dans l'éther comme solvant.

Le tixocortol pivalate est synthétisé à partir de l'acide thiopivalique. Or, l'acide thiopivalique présente une très forte odeur soufrée qui rend son utilisation complexe nécessitant un environnement adapté. Son emploi en synthèse organique est très restreint dans la mesure où il faut disposer d'un site adéquat pour le manipuler. Ces conditions ne sont pas aisément mises en oeuvre d'autant plus pour des génériqueurs qui doivent limiter les coûts de production.

Il existe donc le besoin de trouver une solution pour synthétiser du tixocortol pivalate dans des conditions correctes sans nécessité d'un site adapté.

La présente divulgation propose un sel minéral de l'acide thiopivalique choisi parmi le césium, le potassium et le lithium comme produit intermédiaire pour la synthèse de tixocortol pivalate. Les sels de césium, de potassium et de lithium de l'acide thiopivalique sont très peu odorants et peuvent ainsi être transportés et utilisés de manière simple.

Suivant des variantes préférées mais non limitatives pouvant être alternatives ou cumulatives, l'invention est telle que :
- le sel minéral est un sel de césium,
- le sel de césium de l'acide thiopivalique comprend moins de 1% de pivalate de césium.

Un objet de l'invention est un procédé de synthèse d'un sel minéral de l'acide thiopivalique caractérisé en ce qu'il comprend une étape de réaction dans un solvant organique, tel que défini dans les revendications, entre l'acide thiopivalique et un sel minéral choisi parmi le carbonate de césium et un sel de lithium.

Le procédé de synthèse, selon l'invention, du sel de césium ou de lithium de l'acide thiopivalique présente également l'avantage de générer très peu de sous-produits tels le pivalate de césium ou de lithium. La présence notamment de moins de 1% de ces sous-produits évite la formation d'impuretés type ester pivalate. Préférentiellement, le procédé de synthèse n'implique pas d'alcool. On entend par là qu'il n'y ni ajout, ni formation d'alcool, ni reste d'alcool à l'issue du procédé, ce qui évite le risque de formation notamment de méthylsulfonates d'alkyle à chaine courte connus comme étant des produits génotoxiques. Le procédé selon l'invention et les sels produits sont particulièrement intéressants pour la synthèse de produits à application pharmaceutique.

Suivant l'invention, le procédé de synthèse d'un sel minéral de l'acide thiopivalique est tel que :
- le solvant organique est choisi parmi le méthyl-tétrahydrofurane, le toluène, le tétrahydrofurane, les acétates d'éthyle et d'isopropyle.
- le sel minéral est le carbonate de césium ou un sel de lithium,
- le sel de césium de l'acide thiopivalique peut comprendre moins de 1% de pivalate de césium,
- la réaction a lieu à une température qui peut être comprise entre 20°C et 60°C,

Un autre objet de la divulgation est un produit intermédiaire destiné à la production de tixocortol pivalate, susceptible d'être obtenu par le procédé tel que décrit ci-dessus.

Un autre objet de la divulgation concerne l'utilisation du produit intermédiaire tel que décrit ci-dessus pour la production de tixocortol pivalate.

Un autre objet de la divulgation concerne un procédé de synthèse de tixocortol pivalate à partir d'un sel minéral de l'acide thiopivalique, le sel minéral étant choisi parmi le césium, le potassium et le lithium.

Préférentiellement, le tixocortol pivalate obtenu comporte des traces de césium, avantageusement entre 2 et 6 ppm permettant de marquer le procédé. En outre le sel de césium est préféré pour faciliter sa récupération dans une phase aqueuse.

Suivant des variantes préférées mais non limitatives pouvant être alternatives ou cumulatives, le procédé est tel que :
- il comprend une étape de réaction entre un sel minéral de l'acide thiopivalique et du mésylate d'hydrocortisone dans un solvant organique,
- il comprend ensuite une étape de précipitation et de filtration pour obtenir le tixocortol pivalate,
- le solvant organique est choisi parmi le méthyl-tétrahydrofurane ou le tétrahydrofurane à température ambiante,
- le sel minéral est un sel de césium.

D'autres buts et avantages apparaitront au cours de la description qui suit de l'invention.

Les sels minéraux de l'acide thiopivalique sont particulièrement intéressants car ils sont facilement produits, isolés et transportés pour permettre leur utilisation ultérieure dans des synthèses organiques. Les sels de césium, potassium ou lithium sont préférés notamment pour leur stabilité. Plus particulièrement, le sel de césium et le sel de potassium présentent une réactivité performante sur des dérivés halogénés ou des mésylates ou des tosylates. Le sel de lithium présente des solubilités avantageuses dans certains solvants.

Ces sels de thiopivalate sont préparés par un procédé de synthèse selon l'invention. Ledit procédé comprend une étape de réaction entre l'acide thiopivalique et le carbonate de césium ou un sel de lithium. La réaction est réalisée dans un solvant organique. Ce procédé est facilement industrialisable.

Le rapport molaire entre l'acide thiopivalique et le sel de césium ou de lithium est avantageusement de 1 :1.

Préférentiellement, l'acide thiopivalique utilisé est un acide de qualité commerciale courante, c'est-à-dire de préférence à au moins 97%, plus précisément au moins 99%.

Le sel de césium est préférentiellement un carbonate de césium. Ce sel est facilement accessible. De manière préférée, le carbonate de césium présente une qualité commerciale courante, c'est-à-dire à au moins 97%, plus précisément au moins 99%.

La réaction entre le sel minéral et l'acide thiopivalique se fait avantageusement sous agitation. L'étape de réaction a une durée comprise entre 12 heures et 36 heures. C'est-à-dire que préférentiellement l'agitation est maintenue pendant cette durée.

L'étape de réaction se fait à une température comprise préférentiellement entre 20°C et 60°C, plus préférentiellement entre 20°C et 25°C.

Le solvant organique est non hydroxylé. Avantageusement, ledit solvant est non miscible dans l'eau. Un tel solvant présente l'avantage de limiter la formation de sous-produits notamment l'acide pivalique. Avantageusement, le procédé de synthèse ne faisant pas intervenir d'alcool, l'acide pivalique est en très faible quantité, préférentiellement inférieure à 1%. Un solvant hydroxylé permet une solubilisation de l'eau qui induit la formation d'acide pivalique : c'est notamment le cas avec le THF. D'autre part, dans le cas de réaction sur un ester d'alkyl ou d'arylsulfonique , il y a la formation d'ester sulfonique comme le mésylate ou le tosylate ou le benzylate de méthyle, d'éthyle ou de propyle à fort potentiel génotoxique. Le solvant est choisi parmi le méthyl-tétrahydrofurane, le toluène, le tétrahydrofurane, les acétates d'éthyle et d'isopropyle.

Le tixocortol pivalate obtenu présente une pureté par chromatographie liquide haute performance (HPLC) supérieure ou égale à 98%.

Le tixocortol pivalate se présente après séchage sous forme d'une poudre de couleur sensiblement blanche.

Dans la suite, la présente invention est illustrée plus en détail et plus spécifiquement à l'aide d'exemples sans toutefois en limiter la portée

### Exemple 1 : préparation du thiopivalate de césium dans le MTBE (pas selon l'invention)

L'acide thiopivalique (5 g - 42,3 mmol) est ajouté à une suspension de carbonate de césium (6,9 g - 21,2mmol) dans le MTBE (60 ml). La suspension obtenue est agitée pendant 30 heures à 20-25°C puis la suspension épaisse est filtrée et le solide obtenu lavé par le MTBE (4x 20 ml) puis séché sous vide pour fournir 9,8 g d'un solide blanc (rendement 84%) contenant moins de 1% de pivalate de césium par analyse RMN mais avec la présence d'un peu de carbonate de césium.

### Exemple 2 : préparation du thiopivalate de césium dans le MTBE à 40°C (pas selon l'invention)

La réaction est réalisée comme dans l'exemple 1 mais en utilisant 40 g d'acide thiopivalique pour 55,4 g de carbonate de césium et 480 ml de MTBE. La réaction est terminée après 23 heures d'agitation à 40°C et 1 nuit à 20-25°C. Après filtration du solide, lavage par le MTBE et séchage sous vide, il est obtenu 80,6 g d'un solide blanc (rendement 95%) ne contenant pas de pivalate de césium par analyse RMN mais avec la présence d'un peu de carbonate de césium.

### Exemple 3 : préparation du thiopivalate de césium dans le THF

L'acide thiopivalique (5 g - 42,3 mmol) est ajouté à une suspension de carbonate de césium (6,9 g - 21,2 mmol) dans le tétrahydrofurane (THF) (60 ml). Le mélange réactionnel s'homogénéise rapidement et reste sous agitation 1 nuit à 20-25°C puis est concentré sous vide. Le résidu jaune est repris dans le MTBE (60 ml) et laissé sous agitation la nuit à 20-25°C. La suspension est filtrée et le solide blanc obtenu est séché sous vide pour fournir 10,1 g d'un solide blanc (rendement : 95%) contenant environ 3% de pivalate de césium par RMN.

### Exemple 4 : Préparation du thiopivalate de césium dans le Méthyl-THF

L'acide thiopivalique (5 g - 42,3 mmol) est ajouté à une suspension de carbonate de Césium (6,9 g - 21,2 mmol) dans le 2-méthyl-tétrahydrofurane (Me-THF) (60ml). La suspension obtenue est ensuite agitée pendant 2 heures 30 minutes à 20-25°C. Le précipité est alors filtré et lavé au MTBE (2x 40 ml) et séché sous vide pour fournir une première fraction de 6,95 g d'un solide blanc (rendement 66%) ne contenant pas de pivalate de césium par analyse RMN. Les eaux-mères sont récupérées et filtrées de nouveau pour fournir 1,43 g d'un solide blanc (rendement 13%) contenant environ 0,7% de pivalate de césium par analyse RMN.

### Exemple 5 : préparation du thiopivalate de césium dans le méthyl -THF

L'acide thiopivalique (5 g - 42,3 mmol) est ajouté à une suspension de carbonate de césium (6,9 g - 21,2 mmol) dans le Méthyl-THF (35 ml). La suspension est agitée pendant 7 heures à 20-25°C et se colore en jaune. Après refroidissement à 5°C, le précipité est filtré, lavé au MTBE (4x50 ml) et séché sous vide pour fournir 6,77 g d'un solide blanc (rendement 64%) ne contenant pas de pivalate de césium par analyse RMN.

### Exemple 6 : préparation du thiopivalate de césium dans le mélange Méthyl-THF et MTBE (pas selon l'invention)

L'acide thiopivalique (5 g - 42,3 mmol) est ajouté à une suspension de carbonate de césium (6,9 g - 21,2 mmol) dans le mélange de méthyl-THF (30 ml) et de MTBE (30 ml). La suspension est agitée pendant 53 heures à 20-25°C. Le précipité obtenu est filtré, lavé par le MTBE (3x10 ml) puis séché sous vide pour fournir 8,4 g d'un solide blanc (rendement 79%) ne contenant pas de pivalate de césium par analyse RMN et titrant 98,9%.

### Exemple 7 : préparation du thiopivalate de césium dans le Méthyl-THF

L'acide thiopivalique (153,1 g - 1,27 mol) est ajouté à une suspension de carbonate de césium (206,9 g - 0,635 mol) dans le Méthyl-THF (1785 ml). La suspension est agitée pendant 24 heures à 20-25°C puis le précipité est filtré, lavé par le MTBE (400 ml) puis séché sous vide pour fournir 235,1 g d'un solide blanc (rendement 74%) ne contenant pas de pivalate de césium par analyse RMN. Le filtrat est concentré jusqu'à 1 volume (150 ml environ) puis le MTBE (153 ml) est ajouté et la suspension est agitée pendant 1 heure à 20-25°C puis le précipité est filtré, lavé par le MTBE (1x150 ml) et séché sous vide pour fournir 69,6 g d'un solide blanc cassé (rendement 22%) ne contenant pas de pivalate de césium par analyse RMN. Le rendement global est donc de 96%.

### Exemple 8 : préparation du thiopivalate de césium dans le méthanol (pas selon l'invention)

L'acide thiopivalique (5 g - 42,3 mmol) est ajouté à une suspension de carbonate de césium (6,9 g - 21,2 mmol) dans le méthanol (60 ml). Le mélange réactionnel est agité pendant 1 nuit à 20-25°C puis concentré sous vide. Le résidu jaune est repris dans le MTBE (30 ml) et la suspension tenue sous agitation pendant la nuit. La suspension obtenue est ensuite filtrée et lavée par le MTBE (2 x10 ml) et séchée sous vide pour fournir 10,3 g d'un solide blanc (rendement 97%) contenant environ 0,5% de pivalate de césium par analyse RMN.

### Exemple 9 : Préparation du thiopivalate de césium dans l'acétate d'éthyle

Le procédé est le même que celui décrit à l'exemple 5 mais en remplaçant le méthyl-THF par l'acétate d'éthyle. Après 2 heures d'agitation à 20-25°C, la réaction est complète. Le précipité obtenu est filtré et lavé par le MTBE (3 x10 ml) puis séché sous vide pour fournir 4,5 g d'un solide blanc (rendement 42%) ne contenant pas de pivalate de césium. Le filtrat est concentré à 1 volume puis du MTBE (10 ml) est ajouté. La suspension est agitée pendant 2 heures à 20-25°C et le précipité filtré, lavé par le MTBE (1x10 ml) et séché sous vide pour fournir 2,7 g (rendement 26%) contenant presque 1% de pivalate par analyse RMN . Au total le rendement est donc de 68%.

### Exemple 10 : préparation du thiopivalate de césium dans l'acétate d'isopropyle

L'acide thiopivalique (5 g - 42,3 mmol) est ajouté à une suspension de carbonate de césium (6,8 g - 20,8 mmol) dans l'acétate d'isopropyle (35 ml). La suspension est agitée pendant 8 heures 30 à 20-25°C puis le précipité est filtré, lavé par le MTBE (1x10 ml) et séché sous vide pour fournir 5,8 g d'un solide blanc (rendement 56%) ne contenant pas de pivalate de césium par analyse RMN. Le filtrat est concentré à 1 volume puis le MTBE (5 ml) est ajouté et la suspension agitée pendant 1 heure à 20-25°C puis filtrée et le précipité lavé par le MTBE (1x10 ml) et séché sous vide pour fournir 3,5 g d'un solide blanc (rendement 33%) ne contenant pas de pivalate de césium par analyse RMN. Au total le rendement est donc de 89%.

### Exemple 11 : préparation du thiopivalate de césium dans le méthyl-THF :

L'acide thiopivalique (751,7 g - 6,37 moles) est ajouté en 30 min à une suspension de carbonate de césium (920 g - 3,19 moles) dans le methyl-THF (10 L). Le mélange réactionnel est agité pendant 2 heures et demie à 20-25°C puis un excès de carbonate de césium (124 g - 0,37 mole) est additionné en 3 portions à 1 heure d'intervalle. La suspension est vigoureusement agitée pendant 18 heures environ à 20-25°C puis la suspension est filtrée et le solide lavé par du méthyl-THF (0,5L) puis du MTBE (2,0L). Après séchage sous vide à 50°C pendant 12 heures, ce solide fournit un 1^{er} jet de 1,19 kg de produit attendu (rendement environ 77%). Après concentration de la phase organique jusqu'à un volume résiduel d'environ 1L, du MTBE (2L) est ajouté à la suspension obtenue et après 2 heures d'agitation à 20-25°C, la suspension est filtrée et le solide lavé par le MTBE (0,3L) et le solide obtenu est séché sous vide à 50°C pour fournir un 2éme jet de 0,35 kg. Les 2 jets sont réunis pour donner un total de 1,53 kg (rendement global de 96%) en un produit pur en RMN, titrant **100, 8%** et présentant une teneur en césium résiduel de 6 ppm.

### Exemple 12 : réaction du thiopivalate de césium sur le mésylate d'hydrocortisone (pas selon l'invention)

Dans un réacteur on charge le thiopivalate de césium (620 g - 2,48 mole) et le tétrahydrofurane (1460 ml) et la suspension est agitée à 20-25°C pendant 15 min puis une solution du mésylate d'hydrocortisone (995g - 2,26 mole) dans le tétrahydrofurane (4600 ml) est ajoutée à la suspension en 1heure environ et en restant à 20°C environ.

Après une agitation de 16 heures à cette température, la suspension est traitée par de l'eau (820 ml) et le milieu réactionnel devient limpide. Les insolubles éventuels sont filtrés puis la solution est diluée doucement par de l'eau (11500 ml) pendant 30 min sous agitation et à une température d'environ 10°C. Le produit précipite et après une agitation d'environ 2 heures, la suspension est filtrée et le précipité lavé abondamment par de l'eau (10 x 820 ml). Après séchage en étuve ventilée à 50°C pendant une nuit, 980gr de produit attendu sont obtenus sous forme d'une poudre blanche avec un rendement de 93% et une pureté HPLC de 98,5%.

### Exemple 13: réaction du thiopivalate de potassium sur le mésylate d'hydrocortisone : (pas selon l'invention)

Dans un réacteur on charge le thiopivalate de potassium (0,9 g - 5,76 mmol) et le tétrahydrofurane (40 ml) et la suspension est agitée pendant 15 min à 25°C puis une solution du mésylate d'hydrocortisone (2,5 g - 5,34 mmol) dans le tétrahydrofurane (10 ml) est ajoutée en 10 min en restant à 20-25°C. La suspension obtenue est agitée pendant 30 min à 20-25°C. Le milieu réactionnel est traité par de l'eau (22 ml) en maintenant la température vers 10°C. Le milieu réactionnel passe par une phase limpide puis précipite. Après une agitation de 1 h à 10°C, la suspension est filtrée et le solide obtenu lavé par l'eau (10 x 10 ml) et séché pour donner 2 g de thiopivalate de tixocortol avec un rendement de 81 % et une pureté HPLC de 99,3%

Le sel thiopivalate de potassium a été préparé directement en faisant réagir l'acide thiopivalique (5 g - 43 mmol) sur une solution de potasse (2,4 g - 42,3 mmol) dans l'éthanol absolu (32 ml) à 20-25°C pendant 16 h. Après évaporation du solvant le MTBE (30 ml) est ajouté et la suspension agitée pendant 15 h à 20-25°C puis filtrée pour fournir 5,5 g de thiopivalate de potassium (rendement 84%) sous forme d'un solide blanc présentant une pureté de 97% en RMN.

### Exemple 14 : réaction du thiopivalate de lithium sur le mésylate d'hydrocortisone (pas selon l'invention)

Selon le procédé précédent en utilisant le thiopivalate de lithium (à la place du thiopivalate de potassium) et en opérant dans les mêmes conditions, on obtient le thiopivalate de tixocortol avec un rendement de 80 % et une pureté HPLC de 99%

Le sel thiopivalate de lithium s'obtient selon le procédé de l'exemple 1 en remplaçant le carbonate de césium par le carbonate de lithium (1,56 g -21,2 mmol) et en utilisant le MTBE comme solvant. Le thiopivalate de lithium est obtenu sous forme d'un solide blanc avec un rendement de 80% et une pureté RMN de 98%.

## Revendications

1. Procédé de synthèse d'un sel minéral de l'acide thiopivalique **caractérisé en ce qu'**il comprend une étape de réaction dans un solvant organique entre l'acide thiopivalique et un sel minéral choisi parmi le carbonate de césium et un sel de lithium, le solvant organique étant choisi parmi le méthyl-tétrahydrofurane, le toluène, le tétrahydrofurane, les acétates d'éthyle et d'isopropyle.

2. Procédé selon la revendication précédente dans lequel le sel de césium de l'acide thiopivalique comprend moins de 1% de pivalate de césium.

3. Procédé selon l'une quelconque des deux revendications précédentes dans lequel la réaction a lieu à une température comprise entre 20°C et 60°C.

## Patentansprüche

1. Verfahren zur Synthese eines Mineralsalzes der Thiopivalinsäure, **dadurch gekennzeichnet, dass** es einen Schritt des Reagierens, in einem organischen Lösungsmittel, zwischen der Thiopivalinsäure und einem Mineralsalz, ausgewählt aus Cäsiumcarbonat und einem Lithiumsalz, umfasst, wobei das organische Lösungsmittel aus Methyltetrahydrofuran, Toluol, Tetrahydrofuran, den Ethyl- und Isopropylacetaten ausgewählt ist.

2. Verfahren nach dem vorstehenden Anspruch, wobei das Cäsiumsalz der Thiopivalinsäure mindestens 1 % Cäsiumpivalat umfasst.

3. Verfahren nach einem der zwei vorstehenden Ansprüche, wobei die Reaktion bei einer Temperatur im Bereich zwischen 20 °C und 60 °C stattfindet.

## Claims

1. Method for synthesis of a mineral salt of thiopivalic acid, **characterised in that** it comprises a step of reaction in an organic solvent between the thiopivalic acid and a mineral salt chosen from caesium carbonate and a lithium salt, the organic solvent being chosen from methyl-tetrahydrofuran, toluene and tetrahydrofuran, ethyl and isopropyl acetates.

2. Method according to the previous claim, wherein the caesium salt of the thiopivalic acid comprises less than 1% caesium pivalate.

3. Method according to any one of the two previous claims, wherein the reaction occurs at a temperature of between 20°C and 60°C.
